# EUROPEAN PATENT SPECIFICATION

(11) **EP 0 928 198 B1**
(45) Date of publication and mention of the grant of the patent: **07.07.2004**
(21) Application number: 97927150.9
(22) Date of filing: 29.05.1997
(51) Int. Cl.: A61K 39/10, A61K 39/116, A61K 39/29, C07K 14/235, C07K 14/085, C07K 14/195

(54) **VACCINE COMPOSITION COMPRISING OUTER MEMBRANE PROTEIN FRAGMENTS OF BORDETELLA PERTUSSIS**
BORDETELLA PERTUSSIS AUSSERMEMBRAN PROTEINFRAGMENTE ENTHALTENDE IMPFSTOFFZUSAMMENSETZUNG
COMPOSITION VACCINALE COMPRENANT DES FRAGMENTS PROTEINIQUES DE LA MEMBRANE EXTERIEURE DU BORDETELLA PERTUSSIS

(30) Priority: 03.06.1996 GB 9611501
(43) Date of publication of application: 14.07.1999
(73) Proprietor: GlaxoSmithKline Biologicals S.A., 1330 Rixensart (BE)
(72) Inventor: SLAOUI, Moncef Mohamed, SmithKline Beecham Biol.SA, B-1330 Rixensart (BE); STEPHENNE, Jean, SmithKline Beecham Biol. S.A, B-1330 Rixensart (BE)
(74) Representative: Tyrrell, Arthur William Russell, Dr.
(86) International application number: PCT/EP1997/002956
(87) International publication number: WO 1997/046255

(56) References cited:
- EP-A- 0 437 687
- EP-A- 0 484 621
- WO-A-93/21950
- WO-A-96/34883
- EMSLEY P ET AL: "Structure of Bordetella pertussis virulence factor P.69 pertactin." NATURE (LONDON) 381 (6577). 1996. 90-92. ISSN: 0028-0836, XP002046154

## Description

The present invention relates to new vaccine formulations, comprising a high dose of pertactin, the 69kda outer membrane protein of Bordetella pertussis (hereinafter termed '69K' or '69K antigen'), disclosed in European Patent 0 162 639. Recombinant 69K (P69) has been described by N F Fairweather et al, Symposium On Pertussis (Bethesda), 26-28 September 1990. The invention also relates to a vaccine comprising more than one antigen, optionally a multivalent vaccine, that is: a vaccine for the amelioration or treatment of more than one disease state. The present invention also relates to the production and use of such vaccines in medicine.

It is known that 69K is an important component of acellular pertussis vaccines (Pa vaccines) for the effective prevention of pertussis. 69K-containing vaccines including 'trivalent' vaccines comprising antigens against diphtheria (D), tetanus (T) and pertussis (Pa) have been described in clinical trials conducted in Italy and Sweden and are marketed [for example under the Trade Mark INFANRIX-DTPa (SmithKline Beecham Biologicals)] Typically the pertussis component of such vaccines comprises pertussis toxin (PT), filamentous haemagglutinin (FHA) and 69K. In one such vaccine the amounts of PT.FHA:69K used are 25µg:25µg:8µg per 0.5 ml dose of bulk vaccine.

Other multivalent vaccines comprising 69K are also known, for example vaccines comprising an antigen against hepatitis B and antigens against diphtheria, tetanus and pertussis (Hep B, DTPa) were described in WO 93/24148. In such formulations the quantity of 69K disclosed did not exceed 8 µg per 0 5 ml dose of bulk vaccine.

It has now been found that vaccines containing a high dose of 69K are especially effective in preventing pertussis. Within the limits described herein such vaccines are safe when administered to human subjects and induce rapid protection against B. pertussis infection. In combination vaccines comprising 69K and other antigens it has surprisingly been found that there is no interference, i.e. such vaccines show no loss of immunogenicity, and are effective when administered to humans. In particular the vaccines of this invention are suitable for administration to children. Accordingly the present invention provides a vaccine composition comprising a high dose of 69K antigen. It will be understood that the antigen in the vaccine composition is formulated with a suitable carrier or adjuvant.

By 'high dose of 69K antigen' is meant a dose wherein the concentration of 69K is at least 10µg per 0.5 ml dose of bulk vaccine and is preferably in the range 10µg to 100µg per 0.5 ml dose of bulk vaccine. More preferably the concentration of 69K in the vaccine is in the range 10-50µg, for example about 30µg. A dose of 15-35µg, typically about 20 to 25µg per 0.5 ml dose of bulk vaccine may also advantageously be used.

EP-A-0 437 687 discloses a vaccine containing the outer membrane protein 69kDa from *Bordetella pertussis* in the range of 0.01 - 5 mg/ml as a final concentration of the antigenic protein.

In a further aspect the invention provides a vaccine composition comprising a high dose of 69K (as hereinabove defined) in combination with one or more antigens.

In one aspect such a vaccine can comprise one or more pertussis antigens, such as PT and FHA which are well known in the art. EP-A-0 484 621 discloses the antigen FHA in a *Bordetella pertussis* vaccine in combination with the 69kDa outer membrane protein. Optionally the PT component may be recombinant (for example as described in European Patent Applications EP 0 306 318, EP 0 322 533, EP 0 396 964, EP 0 322 115 and EP 0 275 689) or the PT component may be toxoided, for example as described in EP 0 515 415. See also EP 0 427 462 and WO 91/12020 for the preparation of pertussis antigens. Typically the PT and FHA components will be in the range 10-25µg per 0.5 ml dose of bulk vaccine. In one aspect the ratio of PT:FHA:69K may be approximately 1:1:1.

In a further aspect the vaccine composition of the invention can comprise antigens for prevention of diseases other than pertussis, for example conventional toxoided diphtheria and tetanus antigens. A favoured vaccine composition of the invention is thus DTPa* where Pa* denotes a combination of PT, FHA and high dose 69K as hereinabove defined. It has been surprisingly found that the high dose of 69K is not reactogenic and does not lead to interference with the other components of the DTPa* vaccine composition. Neither does a high dose of 69K substantially reduce the immunogenicity of any of the antigenic components of the multivalent vaccines described hereinbelow.

In yet a further aspect the invention provides a multivalent vaccine comprising a high dose of 69K, particularly Pa* as defined above, in which an antigen against hepatitis B (Hep B) is present (i.e. preferably a Hep B - DTPa* vaccine). Such multivalent vaccines are generally as described in WO 93/24148 except that a high dose of 69K as hereinabove defined is used in the formulation. As described in WO 93/24148 the hepatitis B antigen is preferably hepatitis B surface antigen.

The preparation of Hepatitis B surface antigen (HBsAg) is well documented. See for example, Harford et al in Develop. Biol. Standard 54, page 125 (1983), Gregg et al in Biotechnology, 5, page 479 (1987), EP-A-0 226 846, EP-A-0 299 108 and references therein.

As used herein the expression 'Hepatitis B surface antigen' or 'HBsAg' includes any HBsAg antigen or fragment thereof displaying the antigenicity of HBV surface antigen. It will be understood that in addition to the 226 amino acid sequence of the HBsAg S antigen (see Tiollais et al, Nature, 317, 489 (1985) and references therein) HBsAg as herein described may, if desired, contain all or part of a pre-S sequence as described in the above references and in EP-A- 0 278 940. HBsAg as herein described can also refer to variants, for example the 'escape mutant' described in WO 91/14703. In a further aspect the HBsAg may comprise a protein described as L* in European Patent Application Number 0 414 374, that is to say a protein, the amino acid sequence of which consists of parts of the amino acid sequence of the hepatitis B virus large (L) protein (ad or ay subtype), characterised in that the amino acid sequence of the protein consists of either:
(a) residues 12 - 52, followed by residues 133 - 145, followed by residues 175 - 400 of the said L protein; or
(b) residue 12, followed by residues 14 - 52, followed by residues 133 - 145, followed by residues 175 - 400 of the said L protein.

HBsAg may also refer to polypeptides described in EP 0 198 474 or EP 0 304 578.

Normally the HBsAg will be in particle form. It may comprise S protein alone or may be as composite particles, for example (L*,S) wherein L* is as defined above and S denotes the S-protein of hepatitis B surface antigen.

Preferably the HBsAg will be adsorbed on aluminium phosphate as described in WO93/24148. Other antigens may be adsorbed onto aluminium phosphate or aluminium hydroxide but in some cases satisfactory results will be obtained only if the other antigen is adsorbed on aluminium phosphate. For example in the case of Hib (as described below) it may be appropriate to pre-adsorb the Hib to aluminium phosphate.

Other antigens may included in vaccine of the invention, for example in the Pa* or DTPa* formulations, to provide other multivalent vaccines. Suitable said other antigens may comprise those known in the art to provide protection against Haemophilus influenzae b (Hib) and/or polio (IPV) and/or hepatitis A, as described in WO 93/24148.

A vaccine for the prevention of Haemophilus influenzae b (Hib) infections is based on the capsular polysaccharide (PRP) conjugated with a carrier protein. The polysaccharide is a polymer of ribose, ribitol and phosphate These vaccines are typically presented as plain (ie without adjuvantation) formulations. Although in one case, (Pedvax Hib produce by Merck) a diluent containing aluminium hydroxide is utilised to reconstitute the lyophilised conjugate. Typically the carrier protein is a diphtheria or tetanus toxoid or an outer membrane protein of N.meningitidis. Examples of such conjugate vaccine antigens are disclosed in US 4 365 170, US 4 673 574, EP 208 375, EP 477508 and EP 161 188.

In the case of Hib combination vaccines comprising Pa*, it might be expected that simple mixing of the components would result in a reduction of antibody titres to the polysaccharide (Hib) component.

The inventors have discovered that this reduction can be inhibited if the conjugate antigen is adsorbed on to aluminium phosphate. In contrast, if the antigen is adsorbed on to aluminium hydroxide, there is a complete reduction of antibody titres to the polysaccharide component.

Accordingly in one preferred aspect of the present invention there is provided a vaccine composition comprising high dose 69K (especially Pa* or DTPa*) and a polysaccharide conjugate antigen adsorbed on to aluminium phosphate. Preferably the antigen is capsular polysaccharide (PRP) from Hib conjugated with a carrier protein.

Preferably the carrier protein is either diphtheria, tetanus toxoid or a fragment thereof (advantageously fragment C), diphtheria Crm₁₉₇ protein or an outer membrane protein from a bacteria such as N.meningitidis.

The polysaccharide conjugate may be prepared by any known coupling technique. For example the polysaccharide can be coupled via a thioether linkage. This conjugation method relies on activation of the polysaccharide with 1-cyano-4-dimethylamino pyridinium tetrafluoroborate (CDAP) to form a cyanate ester. The activated polysaccharide may thus be coupled directly or via a spacer group to an amino group on the carrier protein. Preferably, the cyanate ester is coupled with hexane diamine and the amino-derivatised polysaccharide is conjugated to the carrier protein using heteroligation chemistry involving the formation of the thioether linkage. Such conjugates are described in PCT published application WO93/15760 Uniformed Services University.

The conjugates can also be prepared by direct reductive amination methods as described in US 4365170 (Jennings) and US 4673574 (Anderson). Other methods are described in EP-0-161-188, EP-208375 and EP-0-477508.

A further method involves the coupling of a cyanogen bromide activated polysaccharide derivatised with adipic acid hydrazide (ADH) to the protein carrier by carbodiimide condensation. Such conjugation is described in Chu C. et al Infec . Immunity, 1983 245 256.

In a preferred embodiment of the invention the ratio of PRP polysaccharide to carrier protein is modifed from a typical 1:3 to 1:0.3 to 1:2.

WO-A-93/21950 discloses a vaccine comprising DTPa* - Hib. Particular multivalent vaccine compositions within the scope of the invention include a Hep B-DTPa* vaccine, a DTPa*-Hib-Hepatitis B vaccine, and a DTPa*-Hib-Hepatitis B - IPV (inactivated polio) vaccine and a DTPa* - Hepatitis B - IPV vaccine (DTPa*HBIPV).

The above combinations may optionally include a component which is protective against Hepatitis A.

Suitable components for use in such vaccines are already commercially available and details may be obtained from the World Health Organisation. For example the IPV component may be the Salk inactivated polio vaccine. The component affording protection against Hepatitis A is preferably the product known as 'Havrix' (SmithKline Beecham Biologicals) which is a killed attenuated vaccine derived from the HM-175 strain of HAV [see 'Inactivated Candidate Vaccines for Hepatitis A' by F.E. Andre, A Hepbum and E.D'Hondt, Prog Med. Virol. Vol 37, pages 72-95 (1990) and the product monograph 'Havrix' published by SmithKline Beecham Biologicals (1991)]. Conveniently, the Hepatitis B component may comprise the 'S' antigen as already present in the commercial vaccine 'Engerix-B' (SmithKline Beecham Biologicals).

The amount of antigen in each vaccine dose is selected as an amount which induces an immunoprotective response without significant, adverse side effects in typical vaccinees. Such amount will vary depending on which specific immunogens are employed. Generally it is expected that each dose will comprise 10-100µg of total immunogen. Following an initial vaccination, subjects may receive one or two booster injections at about 4 weeks intervals.
In general the vaccine formulations of any aspect of the invention can be prepared as follows. The required Pa* components are adsorbed onto a suitable adjuvant, most especially aluminium phosphate. After allowing time for complete and stable adsorption of the respective components, the different components can be combined under appropriate conditions.

In a preferred aspect of preparing a combined Hepatitis B-containing vaccine composition according to the invention there is provided a method which involves mixing aluminium phosphate-adsorbed HBsAg with one or more aluminium hydroxide or aluminium phosphate-adsorbed antigens.

The following examples illustrate the invention:

### Example 1: Preparation of a vaccine formulation containing purified high dose 69K antigen alone and a safety trial in adult subjects

The 69K-containing vaccine was formulated by standard techniques and a dose of 20 µg of 69K per 0.5 ml dose of bulk vaccine was used.

When the vaccine formulation was tested in adult humans, the results showed that the high dose 69K-containing vaccine was safe.

The details were as follows.

In a double-blind, randomised, placebo-controlled study, 50 healthy adult volunteers with history of vaccination with whole-cell pertussis vaccine in childhood received either a monocomponent pertussis vaccine preparation containing 20µg of 69kDa (20 subjects), a bicomponent preparation with 25 µg PT and 25 µg FHA (20 subjects) or placebo consisting of the vaccine excipients without the antigens (10 subjects) as booster vaccination

The study participants received one of the two study vaccines or a placebo in the present study
Group I: 20 µg 69kDa (monocomponent acellular pertussis vaccine (0.5 ml/dose). Lot 69K1A2
Group II: 25 µg PT/25 µg FHA (bicomponent acellular pertussis vaccine) Lot DPSK 314A2.
Group III placebo consisting of the vaccine excipients without the acellular pertussis components, ie:
   - 1 dose = 0.5 ml
   - 150 mM NaCl
   - 0.5 mg Al+3 (Al(OH)₃)
   - 2.5 mg 2-phenyoxyethanol
   - pH 6.0-7.0

Fifty-five monodose vials of the study vaccines and placebo, coded according to a randomisation list, were distributed.

On days 7, 14 and 28 subjects were examined by the investigator and diary card completion was verified. Post-vaccination blood samples were collected on days 7 and 28

### Results

The 69kDa vaccine was no more reactogenic than the PT/FHA vaccine. Swelling and redness were seen in the study vaccine groups, and not in the placebo group, but never in more than 20% of subject. Pain was reported by 85% of subjects in both the 69kDa group and the PT/FHA group and by 80% of subjects receiving placebo, indicating that pain may be due more to the vaccine excipients than the antigens themselves. A large percentage of reported pain was not spontaneous pain but only pain when pressure was placed on the injection site.

Late-onset or biphasic local reactions were reported by some recipients of the PT/FHA vaccine while local reactions for 69kDa recipients occurred generally within the first 4 days following vaccination and were of short duration. Two severe local reactions of long duration were reported in the PT/FHA group, the cause of which are unclear.

No clinically significant fever was reported. Solicited general symptoms and unsolicited symptoms were seen at low frequencies in all three study groups and are most likely not causally associated with vaccination.

An immune response to 69kDa was seen in 100% of recipients of the antigen with a 77-fold increase in geometric mean titre (GMT) of anti-69kDa antibodies over prevaccination titre by day 28 following vaccination. 90% and 95% ofbicomponent PT/FHA recipients demonstrated an immune response to PT and FHA, respectively, with anti-PT antibody GMT increasing 25-fold and anti-FHA antibodies increasing 80-fold over prevaccination titres.

It may be concluded that the 20 µg dose of 69kDa could be safely administered to adults.

### Example 2: Vaccine formulation comprising Hib polysaccharide conjugated on tetanus toxoid adsorbed onto aluminium phosphate.

### Synthesis of Haemophilus influenzae type B capsular polysaccharide (PRP) Tetanus toxoid (TT) conjugate

### (a) Cyanogen Bromide Coupling

The covalent binding of PRP and TT is carried out by a coupling chemistry developed at the NIH (Chu C. et al (1983), further studies on the immunogenicity of Haemophilus influenzae type b and pneumococcal type 6A polysaccharide protein conjugates. Infec. Immunity, 245-256). The PRP is activated under controlled conditions by cyanogen bromide and derivatised with an adipic hydrazide spacer.

After derivatisation, the activated polysaccharide (PRP-AH) is purified by diafiltration. The coupling of the two purified components (PRP-AH and TT) is effected by carbodiimide condensation The conjugate is then purified by ultrafiltration and gel filtration to remove the reagent and unconjugated PRP and TT.

### Synthesis of PRP-TT Conjugates

### (b) CDAP coupling

30mg of native Hib PRP were dissolved in 6ml 2M NaCl. 225mcl ofCDAP (1 cyano-4-dimethylamino-pyridinum tetrafluoroborate) was added to the polysaccharide solution (from a 100 mg/ml stock solution in acetonitrile) 90 seconds later, 450 mcl of 0.2 M triethylamine was added. The activation was performed at pH 10 0 during 1 minute on ice and minute at room temperature.

90 mg of tetanus toxoid (initial PS/protein ratio of 1/3) were added to the activated polysaccharide and the coupling reaction was performed at room temperature for 1 hour. Then, the reaction was quenched with 3 ml of 1M glycine solution, pH 5.0 for 30 minutes at room temperature and overnight at 4°C.

The conjugate was purified by gel filtration on a sephacryl HR 500 column equilibrated in 0.2M NaCl. The carbohydrate and protein content was determined in each fraction. The conjugate was pooled and sterile filtered (membrane Minisart ⊕ 0.222 γm).

### (c) Adsorption on to aluminium phosphate

To 0.15mg of aluminium phosphate was added 12.5 µg of the polysaccharide conjugate of example 2(a). This was stirred for two hours the pH is adjusted to 5.1. The mixture was left to stand for one day at room temperature and the adsorbed conjugate then left for a further 9 days at 2 to 8 °C. To prepare a freeze dried product the adsorbed product is diluted in lactose (15.75mg) to give a final composition of 25µg polysaccharide/ml and 0.4mg Al/ml and the resulting composition was filled into 0.5ml vials and freezed dried.

To prepare a liquid product the adsorbed conjugate is diluted in water for injection with 150mM NaCl and 5mg/ml phenoxy ethanol to give a final composition of 20 µg polysaccharide/ml and 0.32 mgAl/ml.

### (d) Formulation of a Diphtheria Tetanus and Pertussis (acellular) vaccine with and without hepatitis B

This was done in accordance to the methods of WO 93/24148 (SmithKline Beecham Biologicals). The formulation was made up in accordance with Example 4 below.

### (e) Preparation of a 'low ratio' PRP-TT aluminium phosphate pre-adsorbed conjugate.

The conjugate was prepared in an analogous manner to the example of 2(a), but with reduced amount of Tetanus used (30µg, 60µg) to give a product with Polysaccharide:Protein ratio of 1:1 or 1:2. The conjugate is then adsorbed on to aluminium phosphate according to the method of example 2(c). The final freeze dried preparation contains 12.5 µg of conjugate, 0.15mg ALPO₄, 15.75 mg lactose. This is reconstituted in 0 5ml water for injection prior to use at a pH of 0.1 +/- 0.1.

### Example 3: Immunogenicity of PRP-TT conjugate preadsorbed on aluminium phosphate and combined with DTPa* or DTPa*-Hep B

The Hib conjugate of example 2(a), either plain or pre-adsorbed on AlPO4 (both vaccines were lyophilized) was mixed with DTPa* or DTPa* Hep B no more than 1 hour before injection and the combination was injected in baby rats (1 week of age) by the subcutaneous route at a dose corresponding to 1/20th a human dose (0.5 µg of PRP). The rats were boosted 2 weeks and 4 weeks later and the serum was collected after each immunization to measure anti-PRP antibodies. Controls included the Hib vaccines (adsorbed or not on AlPO4) reconstituted in saline.

Groups of 10 randomized baby rats (1 week of ago-OFA strain) were immunized 3 times subcutaneously at 0-14-28 days with 1/20th human dose of Hib vaccine, alone or combined with DTPa* or DTPa* HepB (1/20th a human dose). The reconstitution of the lyophilised Hib vaccine with saline or combinations (DTPa* or DTPa* Hep B) was done less than 1 hour before immunization.

The rats were bled under anesthesia at 14-28-42 and 56 days. The anti-PRP antibodies were measured by ELISA in individual sera and the titers were expressed in γ/ml using a calibrated reference. The GMT was calculated for each group and for each time point. The 95% confidence limits were calculated for the titers obtained after that third immunization.

The adsorption of Hib conjugate on AlPO4 does not modify its immunogenicity some anti-PS were produced after the second dose and a good booster effect is shown after the third dose as seen in human babies.

### Example 4: Preparation of a Hep B- DTPa* vaccine

The formulation was prepared exactly as in Example 5 of WO93/24148 except that 20 µg of 69K was used to make up a 0.5 ml dose of bulk vaccine.

The procedure may be repeated as desired to make up vaccines comprising between 10 and 50µg of 69K per 0 5ml dose of bulk vaccine.

### Example 5: Performance of a Hep B- DTPa* vaccine and other multivalent vaccines in a mouse model.

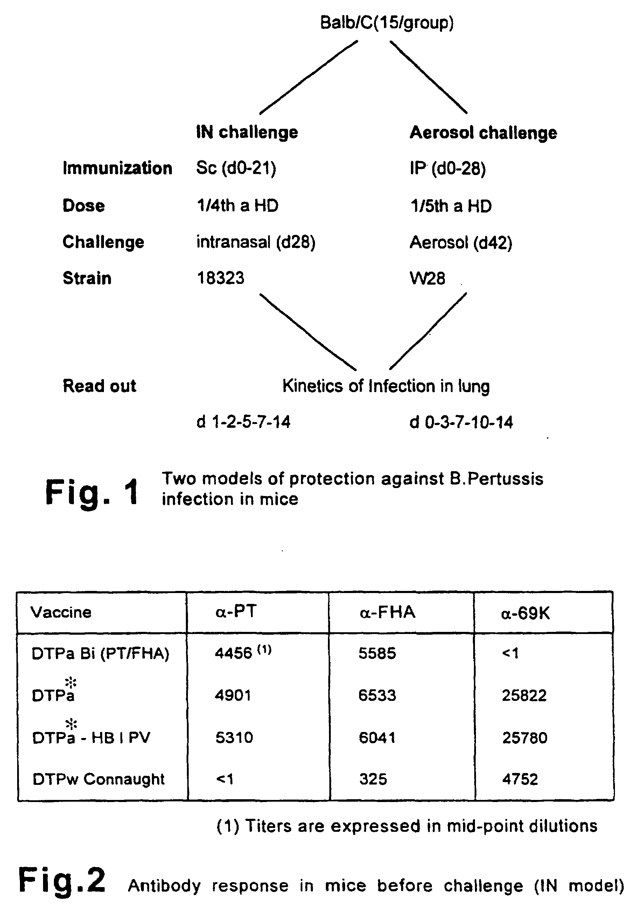

Groups of Balb/C mice (15 per group) are challenged with B pertussis either using intranasal (IN) challenge or aerosol challenge as indicated in Figure 1

The vaccines tested in this challenge may be:
- 'DTPa Bi', in which PaBi denotes PT/FHA with no 69K present
- DTPa*, in which Pa* as is as defined herein, that is PT/FHA/69K with high dose 69K present
- DTPa* HB IPV, that is DTPa* with hepatitis B surface antigen (S-antigen) and IPV present; and
- a whole cell (DTPw) pertussis vaccine (e.g. that manufactured by Connaught)

The 69K-containing formulations may be tested with and without Hib present.

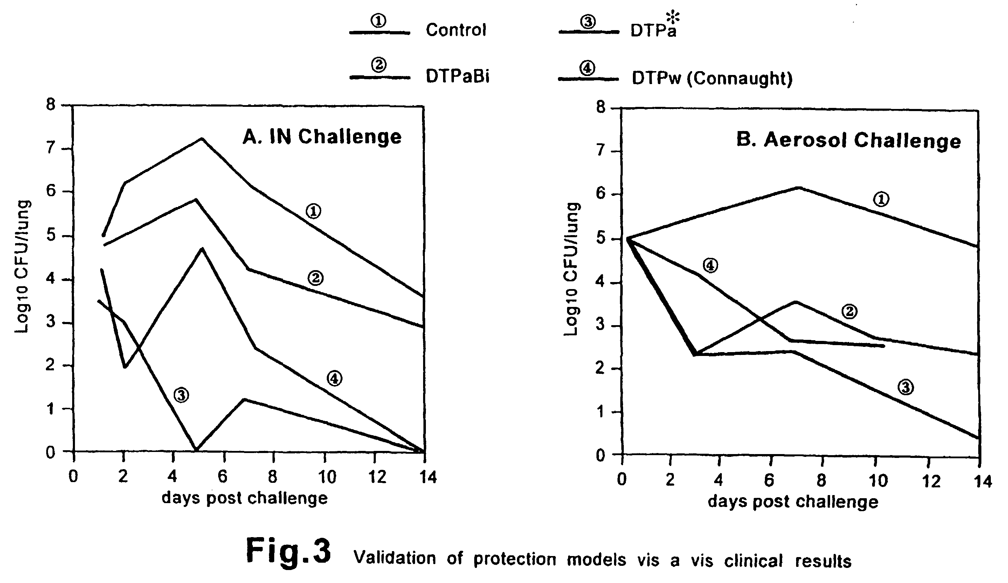

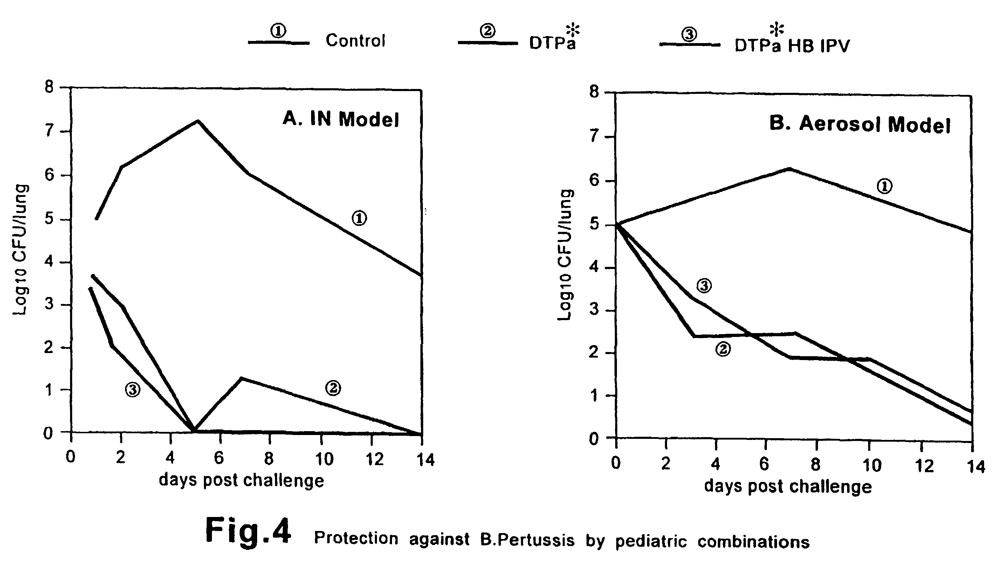

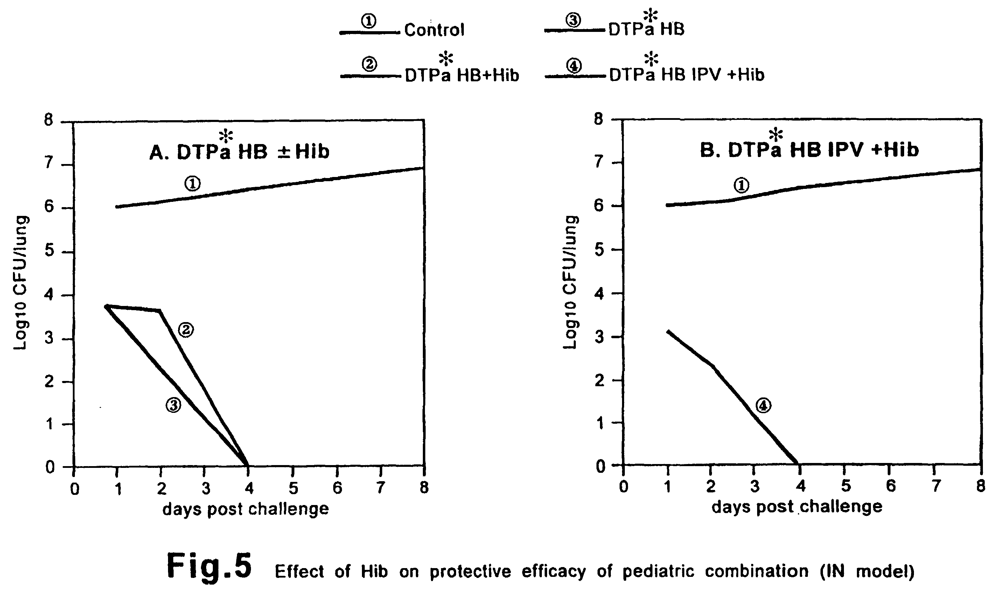

Figures 2 to 5 show the results that may be typically obtained with a dose of 69K in the range 10-25µg per 0.5 ml of bulk vaccine and other vaccine compositions adapted appropriately so as to provide a proper comparison The results that can be obtained demonstrate that the high dose 69K-containing vaccine is superior to the others and, surprisingly, can cause no interference. These data show that 69K is the key protective antigen in vaccines against B pertussis.

### Example 6 : Effects of 69K on protection against B pertussis infection in intranasal challenge model in mice

Groups of 15 to 30 Balb/C mice (females, 5 weeks old) were immunised SC with 1/4th a human dose.

The mice were boosted 3 weeks later with the same dose. One week after the booster, blood samples were taken for antibody evaluation and the mice were challenged by instillation of 50 µl bacterial suspension into the nostrils under light anaesthesia (+/-5.10⁶ cfu/50µl strain 18323, log phase). Five mice were killed at 5 different times (d1,d2, d4,d8,d14) after challenge and the lungs were removed aseptically and homogenised individually in 2 ml of casamicoacid (1% in physiological saline). 100µl of 4 serial 10 fold dilutions were cultured on Bordet-Gengou agar plates for 6 days at 37°C. The number of colony forming units (cfu) was determined for each lung (lower level of detection is 20 cfu/lung). The arithmetic mean of the log 10 of cfu/lung and the standard deviation was calculated for each time point.

The clearance of bacteria was evaluated by calculating the difference (log cfu control mice minus log cfu immunised mice) at day 4,8 and 14. For day 14 the number of protected mice (< 20 cfu/lung) was recorded. The data are shown in the Table below.

| **Vaccine** | | **Lung colonisation (log CFU/lung)** | | ***Protection*** ***at day 14*** |
|---|---|---|---|---|
| | | ***Day 4*** | ***Day 8*** | |
| Control | | 7.63 ± 0.38 | 7.34 ± 0.32 | 0/5 |
| 69K alone (µg) | 0.5 | 5.68 ± 3.22 | 6.56 ± 0.18 | 0/5 |
| | 2 | 6.02 ± 0.57 | 6.52 ± 0.23 | 0/4 |
| | 8 | 4.73 ± 0.16 | 4.86 ± 1.17 | 0/5 |
| | 32 | 4.36 ± 0.86 | 4.75 ± 0.53 | 0/5 |
| PT + FHA + 69k (µg) | 0 | 5.20 ± 1.06 | 4.36 ± 0.88 | 2/3 |
| | 0.5 | 3.14 ± 0.76 | 1.48 ± 2.03 | 3/4 |
| | 2 | 1.85 ± 0.98 | 1.67 ± 0.75 | 4/5 |
| | 8 | 2.74 ± 1.58 | 0.96 ± 1.52 | 5/5 |
| | 32 | 0.80 ± 1.12 | 0.63 ± 1.41 | 5/5 |
| Trivalent DTPa | | 2.34 ± 1.31 | 0.88 ± 1.21 | 5/5 |
| (containing 8 µg 69K; 25µg FHA; 25µg of PT; 25Lf of D toxoid and 10 Lf of T toxoid) | | | | |
| | | | | |
| | | | | |

## Claims

1. A vaccine composition comprising a high dose of the 69kDa outer membrane protein of B pertussis, in which the dose is in the range 10-100ug per 0.5 ml of bulk vaccine and which additionally comprises the pertussis antigens PT and FHA, diphtheria (D) and tetanus (T) antigens and hepatitis B antigen, together with a suitable carrier or adjuvant.

2. A vaccine composition as claimed in claim 1 in which the hepatitis B antigen is the hepatitis B Surface antigen.

3. A vaccine composition as claimed in claim 1 or claim 2 which additionally comprises an Hib type B capsular polysaccharide conjugate.

4. A vaccine composition as claimed in claim 3 in which the polysaccharide conjugate is a tetanus toxoid protein conjugate (PRP-TT).

5. A vaccine composition as claimed in claim 4 in which the polysaccharide to protein ratio is 1:1 or 1:2.

6. A vaccine composition as claimed in any preceding claim which additionally comprises inactivated polio vaccine.

7. A vaccine composition as claimed in any preceding claim which additionally comprises an inactivated hepatitis A vaccine.

8. A vaccine composition as claimed in claim 7 in which the hepatitis A vaccine is derived from the HM-175 strain.

9. A vaccine composition as claimed in any preceding claim in which the adjuvant comprises aluminium phosphate.

## Patentansprüche

1. Impfstoffzusammensetzung, die eine hohe Dosis des 69 kDa äußeren Membranproteins von B. pertussis umfaßt, worin die Dosis im Bereich von 10-100 µg pro 0,5 ml Impfstoffmasse ist, und die zusätzlich die Pertussis-Antigene PT und FHA, Diphtheria-(D)- und Tetanus-(T)-Antigene und Hepatitis B-Antigen zusammen mit einem geeigneten Träger oder Hilfsstoff umfaßt.

2. Impfstoffzusammensetzung gemäß Anspruch 1, worin das Hepatitis B-Antigen das Hepatitis B-Oberflächenantigen ist.

3. Impfstoffzusammensetzung gemäß Anspruch 1 oder 2, die zusätzlich ein Hib-Typ B Kapselpolysaccharid-Konjugat umfaßt.

4. Impfstoffzusammensetzung gemäß Anspruch 3, worin das Polysaccharid-Konjugat ein Tetanus-Toxoid Protein-Konjugat (PRP-TT) ist.

5. Impfstoffzusammensetzung gemäß Anspruch 4, worin das Polysaccharid-zu-Protein-Verhältnis 1:1 oder 1:2 ist.

6. Impfstoffzusammensetzung gemäß jedem vorhergehenden Anspruch, die zusätzlich inaktivierten Polio-Impfstoff umfaßt.

7. Impfstoffzusammensetzung gemäß jedem vorhergehenden Anspruch, die zusätzlich einen inaktivierten Hepatitis A-Impfstoff umfaßt.

8. Impfstoffzusammensetzung gemäß Anspruch 7, worin der Hepatitis A-Impfstoff aus dem HM-175-Stamm stammt.

9. Impfstoffzusammensetzung gemäß jedem vorhergehenden Anspruch, worin der Hilfsstoff Aluminiumphosphat umfaßt.

## Revendications

1. Composition vaccinale comprenant une dose élevée de la protéine de membrane externe de B. pertussis, dans laquelle la dose est dans la plage de 10 à 100 µg par 0,5 ml de vaccin brut et qui comprend, de plus, les antigènes de pertussis PT et FHA, des antigènes de la diphtérie (D) et du tétanos (T) et un antigène de l'hépatite B, conjointement avec un véhicule ou adjuvant adapté.

2. Composition vaccinale selon la revendication 1 dans laquelle l'antigène de l'hépatite B est l'antigène de surface de l'hépatite B.

3. Composition vaccinale selon la revendication 1 ou la revendication 2 qui comprend, de plus, un conjugué de polysaccharide capsulaire de Hib de type B.

4. Composition vaccinale selon la revendication 3 dans laquelle le conjugué de polysaccharide est un conjugué de protéine d'anatoxine de tétanos (PRP-TT).

5. Composition vaccinale selon la revendication 4 dans laquelle le rapport du polysaccharide à la protéine est de 1:1 ou 1:2.

6. Composition vaccinale selon l'une quelconque des revendications précédentes qui comprend, de plus, un vaccin de la poliomyélite inactivé.

7. Composition vaccinale selon l'une quelconque des revendications précédentes qui comprend, de plus, un vaccin de l'hépatite A inactivé.

8. Composition vaccinale selon la revendication 7 dans laquelle le vaccin de l'hépatite A est dérivé de la souche HM-175.

9. Composition vaccinale selon l'une quelconque des revendications précédentes dans laquelle l'adjuvant comprend du phosphate d'aluminium.
